(19) Europäisches Patentamt
European Patent Office
Office européen des brevets

(11) Veröffentlichungsnummer: **0 217 734 B1**

# (12) EUROPÄISCHE PATENTSCHRIFT

(45) Veröffentlichungstag der Patentschrift: **27.11.91**

(51) Int. Cl.[5]: **C07D 457/12**, A61K 31/48

(21) Anmeldenummer: **86730146.7**

(22) Anmeldetag: **18.09.86**

Die Akte enthält technische Angaben, die nach dem Eingang der Anmeldung eingereicht wurden und die nicht in dieser Patentschrift enthalten sind.

## (54) 12- und 13-Brom-Ergolinderivate.

(30) Priorität: **19.09.85 DE 3533675**

(43) Veröffentlichungstag der Anmeldung:
**08.04.87 Patentblatt 87/15**

(45) Bekanntmachung des Hinweises auf die Patenterteilung:
**27.11.91 Patentblatt 91/48**

(84) Benannte Vertragsstaaten:
**AT BE CH DE FR GB IT LI LU NL SE**

(56) Entgegenhaltungen:
**EP-A- 0 048 695**
**CH-A- 584 220**
**DE-A- 3 445 784**
**EP-A- 0 056 358**
**DE-A- 3 309 493**
**DE-B- 2 330 912**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Sauer, Gerhard, Dr.**
**Königsbacher Zeile 41A**
**W-1000 Berlin 28(DE)**
Erfinder: **Heindl, Josef, Dr.**
**Eitel-Fritz-Strasse 40**
**W-1000 Berlin 38(DE)**
Erfinder: **Schröder, Gertrud, Dr.**
**Theodor-Francke-Strasse 3**
**W-1000 Berlin 42(DE)**
Erfinder: **Wachtel, Helmut, Dr.**
**Asternplatz 2**
**W-1000 Berlin 45(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 12- und 13-Brom-Ergolinderivate, ein Verfahren zu ihrer Herstellung und ihre Verwendung als Arzneimittel. Die neuen 12- und 13-Brom-Ergoline können auch als Zwischenprodukte zur Herstellung neuer pharmakologisch wirksamer Ergolinderivate verwendet werden.

Die erfindungsgemäßen 12- und 13-Brom-Ergolinderivate haben die allgemeine Formel I

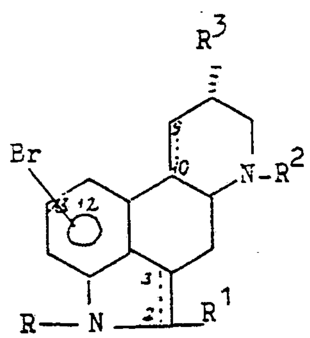

(I)

worin

R ein Wasserstoffatom oder eine Acetylgruppe,

$R^1$ ein Wasserstoffatom, Brom oder eine $C_{1-2}$-Alkylthiogruppe, wobei R und $R^1$ nicht gleichzeitig ein Substituent sein kann,

$R^2$ eine $C_{1-4}$ - Alkylgruppe,

$R^3$ eine NH-CO-$NEt_2$-Gruppe oder eine NH-CS-$NEt_2$-Gruppe bedeutet und

$C_9 \overline{---} C_{10}$ und $C_2 \overline{---} C_3$ eine CC-Einfach- oder eine C=C-Doppelbindung bedeuten, wobei das Wasserstoffatom in 10-Stellung α-ständig ist, wenn $C_9 \overline{---} C_{10}$ eine CC-Einfachbindung ist und das Wasserstoffatom in 3-Stellung α-ständig oder β-ständig ist, wenn $C_2 \overline{---} C_3$ eine CC-Einfachbingung ist und falls Br in 12-Stellung steht, $R^1$ Wasserstoff bedeutet und falls Br in 2-Stellung steht $C_2 \overline{---} C_3$ eine C=C-Doppelbindung bedeutet und deren Säureadditionssalze.

Niedere Alkylreste $R^2$ sind solche mit bis zu 4 C-Atomen, z.B. Methyl, Äthyl, n-Propyl, Isopropyl, n-Butyl, Isobutyl und tert. Butyl.

Die Alkylmercaptan- und Acyl-Schutzgruppen in R und $R^1$ leiten sich von Kohlenwasserstoffresten mit bis zu 2-C-Atomen ab, wobei die Acetyl- und die Methylmercaptangruppe bevorzugt ist.

Die Salze der erfindungsgemäßen Verbindungen der Formel I sind Säureadditionssalze und leiten sich von üblicherweise verwendeten Säuren ab. Solche Säuren sind zum Beispiel anorganische Säuren, wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäuren, Hydroxyalkancarbonsäuren oder Alkendicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat, Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, β-Hydroxybutyrat, Glycollat, Malat, Tartrat, Methansulfonat, Propansulfonat, Naphthalin-1-sulfonat oder Naphthalin-2-sulfonat.

Aus EP-A-056356, DE-A-3309 493 und DE-A-3445784 sind Ergolinderivate bekannt, die in 2-Stellung bromiert und in 12- oder 13-Stellung mit einer Aminogruppe oder einer Nitrogruppe substituiert sein können.

Im Vergleich zu diesen Ergolinen und zu Tergurid (DE-PS-2238540) besitzen die erfindungsgemäßen Verbindungen der Formel I eine stärkere bzw. mindestens gleich starke zentrale $\alpha_2$ -Rezeptorblockierende Wirkung bei schwächeren bzw. fehlenden antidopaminergen Effekten. Dies Wirkprofil läßt die Verbindungen als wertvolle Substanzen zur Behandlung von psychischen Störungen des depressiven Formenkreises erscheinen. Die antidepressive Wirkung der erfindungsgemäßen Verbindungen beruht auf einer zentralen $\alpha_2$ Rezeptorblockade, die eine vermehrte Noradrenalinfreisetzung im Gehirn bewirkt und darüber den antidepressiven Effekt zur Folge hat.

Die zentrale $\alpha_2$-Rezeptorblockade wurde in einem Interaktionstest mit dem $\alpha_2$-Rezeptoragonist Clonidin an Mäusen nach einmaliger i.p. Vorbehandlung dargestellt (Parameter : Aufhebung der durch Clonidin 0,1 mg/kg i.p. verursachten Hypothermie). Männliche NMRI-Mäuse wurden mit verschiedenen Dosen von 1.1-Diethyl-(6-methyl-8$\alpha$-ergolinyl)-harnstoff (TDHL) bzw. 12- oder 13-bromierten Ergolinylharnstoffen, die selbst nicht die Thermoregulation der Versuchstiere beeinflussen, bzw. mit Trägermedium vorbehandelt. 30 Minuten später erhielten alle Tiere Clonidin 0.1 mg/kg i.p. 60 Minuten nach Prüfsubstanz bzw. Trägermedium (= 30 Minuten nach Clonidin) wurde die Rektaltemperatur mit Hilfe einer Thermosonde gemessen. Während die mit Trägermedium vorbehandelten Mäuse eine Hypothermie aufwiesen, war an mit TDHL bzw. 12- oder 13-Brom-Ergolinylharnstoffen vorbehandelten Tieren der körpertemperatursenkende Effekt des Clonidin dosisabhängig aufgehoben. Wie aus Tabelle 1 ersichtlich, war der clonidinantagonistische Effekt nach 13-Br-TDHL in der Dosierung 0,39 mg/kg, und nach 13-Br-2,3-Dihydro-TDHL wie auch bei TDHL in der Dosierung 1,56 mg/kg statistisch signifikant.

Die zentrale Dopaminrezeptorblockade wurde in einem Interaktionstest mit dem Dopaminrezeptoragonisten Apomorphin an Mäusen nach einmaliger i.p.-Vorbehandlung dargestellt. (Parameter : Aufhebung der durch Apomorphin 5 mg/kg i.p. verursachten Hypothermie). Das weitere Vorgehen glich dem bei der zentralen $\alpha_2$-Rezeptorblockade dargestellten Verfahren.

Wie aus Tabelle 2 ersichtlich, war der apomorphinantagonistische Effekt nach TDHL in der Dosierung 3.13 mg/kg statistisch hochsignifikant. Nach 13-Br-TDHL trat ein statistisch signifikanter, quantitativ jedoch schwächerer Effekt in der Dosierung 1.56 mg/kg auf.

Die erfindungsgemäßen Verbindungen zeigen auch blutdrucksenkende Wirkung und eignen sich daher als Arzneimittel zur Hochdrucktherapie.

Ferner eignen sich die erfindungsgemäßen Verbindungen als Zusatz zu Neuroleptika zur Behandlung von Psychosen des schizophrenen Formenkreises insbesondere bei chronischen Schizophrenien mit negativen klinischen Symtomen.

Tabelle 1

Antagonistische Wirkung der Vorbehandlung (30 min. i.p.) mit verschiedenen Dosen von 13-bromierten Ergolinylharnstoffen auf die durch Clonidin (0.1 mg/kg i.p.) ausgelösten Hypothermie an Mäusen. Die Rektaltemperatur der Versuchstiere wurde 30 min. nach Clonidin (= 60 min. nach Prüfsubstanz) gemessen (x : p <0.05, xx : p <0.01 vs Kontrolle; Varianzanalyse/Dunnett-Test)

| Substanz | | Rektaltemperatur [°C](Mittelwert ± S.E.M.) Prüfsubstanzdosis [mg/kg] | | | | | | | |
|---|---|---|---|---|---|---|---|---|---|
| | n | Kontrolle | 0.05 | 0.1 | 0.2 | 0.39 | o.78 | 1.56 | 3.13 |
| TDHL | 8 | 33.1 ± 0.2 | - | - | - | 33.6 ± 0.2 | 33.7 ± 0.3 | 34.1 ± 0.2xx | 34.7 ± 0.3xx |
| 13-Br-TDHL | 8 | 33.2 ± 0.3 | 33.7 ± 0.2 | 32.9 ± 0.2 | 33.8 ± 0.1 | 34.2 ± 0.3x | 34.5 ± 0.3xx | 36.1 ± 0.2xx | - |
| 13-Br-2,3-Dihydro-TDHL | 8 | 34.1 ± 0.2 | - | - | - | 34.3 ± 0.2 | 34.5 ± 0.2 | 36.0 ± 0.4xx | 36.4 ± 0.4xx |

Tabelle 2 :

Antagonistische Wirkung der Vorbehandlung ( 30 min. i.p.) mit verschiedenen Dosen von 13- Brom-Ergolinylharnstoffen auf die dadurch Apomorphin ( 5 mg/kg i.p. ) ausgelöste Hypothermie an Mäusen. Die Rektaltemperatur der Versuchstiere wurde 30 min. nach Apomorphin ( = 60 min. nach Prüfsubstanz ) gemessen (x : $p < 0.05$, xx : $p < 0.01$ vs Kontrolle; Varianzanalyse/Dunnett-Test).

| Substanz | | Rektaltemperatur [°C](Mittelwert ± S.E.M.) Prüfsubstanzdosis [mg/kg] | | | | | | | | |
|---|---|---|---|---|---|---|---|---|---|---|
| | n | Kontrolle | 0.05 | 0.1 | 0.2 | 0.39 | 0.78 | 1.56 | 3.13 | 6.25 |
| TDHL | 8 | 32.5 ± 0.4 | - | - | - | - | 33.9 ± 0.5 | 33.8 ± 0.4 | 35.1 ± 0.5xx | 35.5 ± 0 |
| 13-Br-TDHL | 8 | 32.6 ± 0.2 | 32.5 ± 0.2 | 32.2 ± 0.2 | 32.6 ± 0.4 | 33.0 ± 0.3 | 33.3 ± 0.2 | 33.5 ± 0.3x | - | - |

Die Herstellung der erfindungsgemäßen Verbindungen erfolgt nach an sich bekannten Methoden. Beispielsweise gelangt man zu den Verbindungen der allgemeinen Formel I, indem man eine Verbindung der allgemeinen Formel II

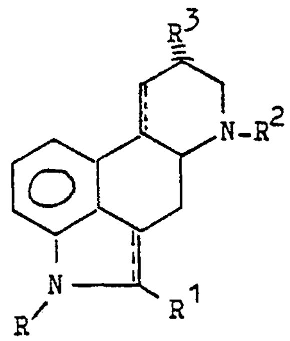

(II)

worin
R, $R^2$, $R^3$ und C---C die obige Bedeutung haben und $R^1$ Wasserstoff oder eine $C_{1-2}$-Alkylthiogruppe bedeutet, mit einem Bromierungsmittel umsetzt und anschließend gegebenenfalls in Verbindungen der allgemeinen Formel I mit einer $C_2=C_3$-Doppelbindung 2-Brom oder eine Alkylthiogruppe abspaltet und gewünscht enfalls die $C_2=C_3$ Doppelbindung hydriert oder in Verbindungen der allgemeinen Formel I mit einer $C_2$-$C_3$-Einfachbindung eine Acylgruppe abspaltet und gewünschtenfalls zur $C_2=C_3$-Doppelbindung dehydriert und anschließend gewünschtenfalls den Harnstoff in den Thioharnstoff überführt und gegebenenfalls das Säureadditionssalz bildet.

Die Bromierung wird in einem inerten Lösungsmittel wie z.B. chlorierten Kohlenwasserstoffen wie Chloroform, Methylenchlorid oder protischen Lösungsmitteln wie wässrige Essigsäure, Eisessig oder Ethern wie Tetrahydrofuran, Dioxan, Diisopropylether usw. in einem Temperaturbereich von - 20 °C bis 80 °C bevorzugt bei Raumtemperatur durchgeführt. Als Bromierungsmittel dienen elementares Brom bzw. Bromierungs-Reagenzien wie Pyridinhydrobromid-per-bromid, Pyrrolidonhydro-perbromid u.a..

Die Abspaltung der Alkylthiogruppe erfolgt beispeilsweise indem man mit einem Reduktionsmittel wie Natriumborhydrid in Trifluoressigsäure bei einer Reaktionstemperatur von - 40 °C bis + 20 °C umsetzt und das Reaktionsprodukt gegebenenfalls in einem inerten Lösungsmittel wie beispielsweise Alkoholen wie Methanol, Ethanol oder Ethern wie Dioxan und Tetrahydrofuran löst und anschließend mit einer Base wie beispielsweise wässriger Alkalihydroxyd-Lösung wie wässriger Kali- und Natriumlauge oder Alkalialkoholaten wie Natriumethylat und Natriummethylat versetzt.

Vergrößert man die Menge an eingesetztem Natriumborhydrid (ca. 2 molar) und erhöht die Reaktionszeit auf ca. 5 Stunden, so wird unter Abspaltung der Mercaptangruppe gleichzeitig die $C_2=C_3$-Doppelbindung hydriert.

Die Abspaltung von Brom in 2-Stellung wir zweckmäßigerweise mit Natriumborhydrid und einem Cobaltsalz beispielsweise Cobaltchlorid oder Cobaltsulfat in protischen Lösungsmitteln wie zum Beispiel Alkoholen wie Methanol, Ethanol, Isopropanol, Wasser oder Mischungen derselben bei Temperaturen von - 20 °C bis + 50 °C vorgenommen.

Die Abspaltung des Acylrestes erfolgt in einem inerten Lösungsmittel wie z.B. chlorierten Kohlenwasserstoffen, Alkoholen, Äthern, Wasser u.a. bei Temperaturen zwischen 0 °C bis 100 °C unter Verwendung von anorganischen und organischen Basen wie KOH, NaOH, Hydrazin, Na-methylat, K-tert.-butylat usw.

Die Abspaltung kann auch in Gegenwart von Säuren erfolgen, bevorzugt anorganischen Säuren wie z.B. HCl, $H_2SO_4$ u.a.

Die Einführung der $C_2=C_3$-Doppelbindung erfolgt nach an sich bekannten Methoden wie z.B. durch Dehydrierung mit $MnO_2$ (DOS 3309493) oder tert. Butylhypochlorit (DOS 3445784.4).

Die Überführung der 8α-Harnstoffderivate in die entprechenden Thioharnstoffderivate erfolgt durch Umsetzung mit Phosphoroxychlorid und einem Thiolierungsmittel nach der deutschen Patentanmeldung P. 35 28 576.1.

Alle Reaktionen werden üblicherweise unter Inertgasatmosphäre vorgenommen wie z.B. Argon oder Stickstoff.

Zur Bildung von Salzen werden die Verbindungen der allgemeinen Formel I in wenig Methanol oder Methylenchlorid gelöst und mit einer konzentrierten Lösung der entsprechenden Säure in Methanol bei Raumtemperatur versetzt.

Zur Verwendung der erfindungsgemäßen Verbindungen als Arzneimittel werden diese in die Form eines pharmazeutischen Präparats gebracht, das neben dem Wirkstoff für die enterale oder parentale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs, Stabilisierungs-, Netzmittel oder Emulgatoren, Salze zur Veränderung des osmotischen Drucks oder Puffer.

Die Herstellung der Ausgangsverbindungen ist bekannt oder erfolgt nach bekannten Methoden.

Die nachfolgenden Beispiele sollen das erfindungsgemäße Verfahren erläutern.

Beispiel 1

3-(13-Brom-6-methyl-2-methylthio-8α-ergolinyl)-1.1-diethylharnstoff

Man löst 11,2 g 3-(6-Methyl-2-methylthio-8α-ergolinyl)-1,1-diethyl-harnstoff (29 mMol) in 400 ml Methylenchlorid p.a. und tropft bei Raumtemperatur 1,45 ml Brom (29 mMol), gelöst in 100 ml Methylenchlorid p.a. innerhalb von 20 Minuten zu. Nach 15 Minuten Rühren wird das Lösungsmittel im Vakuum abdestilliert, der Rückstand in Methylenchlorid und Methanol aufgenommen und durch Zugabe von Essigester mit wenig Diisopropylether und Abziehen der leichtflüchtigen Lösungsmittel kristallisiert.
Ausbeute 12,6 g (93 % d. Th.)
$[\alpha]_D$ = -2 ° (0,5 % in Methanol)

Beispiel 2

3-(13-Brom-6-methyl-8α-ergolinyl)-1.1-diethyl-harnstoff

10,74g(23 mMol) 3-(13-Brom-6-methyl-2-methylthio-8α-ergolinyl)-1,1-diethyl-harnstoff löst man unter Argon in 200 ml Trifluoressigsäure. Bei einer Temperatur von - 15 °C gibt man in 8 Portionen a 0,5 g Natriumborhydrid-Tabletten dazu. Nach 1 ½ Stunden Reaktionszeit bei - 15 °C gießt man die Mischung auf 500 ml Eis, alkalisiert die Lösung vorsichtig mit 25 %iger Ammoniaklösung und extrahiert mit Methylenchlorid. Die organischen Phasen werden über Magnesiumsulfat getrocknet und eingedampft. Das Rohproduckt wird in 100 ml Methanol p.a. unter Argon gelöst und mit 50 ml 7n Kalilauge versetzt. 2 Stunden rührt man bei Raumtemperatur bis zur vollständigen Umsetzung, dann gießt man zur Aufarbeitung auf 150 ml Eis und extrahiert mit Methylenchlorid. Die organischen Phasen werden mit Wasser gewaschen und über Magnesiumsulfat getrocknet. Das eingeengte Rohprodukt wird an 1,4 kg Kieselgel mit Methylenchlorid/Methanol im Verhältnis 97 : 3 chromatographiert. Dabei werden 6,0 g isoliert, die man zur vollständigen Reinigung aus Essigester/Diisopropylether kristallisiert. Hierbei werden 5,44 g 3-(13-Brom-6-methyl-8α-ergolinyl)-1,1-diethyl-harnstoff gewonnen (56 % Ausbeute).
$[\alpha]_D$ = - 9,6 ° (0,5 % in $CH_3OH$)
$[\alpha]_D$ = - 6.4 ° (0,5 % in $CH_3OH$/Pyridin)

Beispiel 3

3-(13-Brom-6-n-propyl-8α-ergolinyl)-1,1-diethyl-harnstoff

Analog Beispiel 1 und Beispiel 2 wird aus 3-(2-Methylthio-6-n-propyl-8α-ergolinyl)-1,1-diethyl-harnstoff das 3-(13-Brom-6-n-propyl-8α-ergolinyl)-1,1-diethyl-harnstoff in 42 % Ausbeute dargestellt.

Beispiel 4

3-(13-Brom-2,3-dihydro-6-methyl-8α-ergolinyl)-1,1-diethyl-harnstoff

Aus dem 3-(13-Brom-6-methyl-2-methylthio-8α-ergolinyl)-1,1-diethyl-harnstoff läßt sich analog Beispiel 2 durch Verdoppelung der Natriumborhydridmenge und Verlängerung der Reaktionszeit auf 5 Stunden 3-(13-Brom-2,3-dihydro-6-methyl-8α-ergolinyl)-1,1-diethyl-harnstoff in einer chromatographischen Ausbeute von 52 % darstellen. Die Substanz kristallisiert aus Essigester,$[\alpha]_D$ = + 19 ° (0,5 % in Chloroform).

Beispiel 5

3-(13-Brom-6-methyl-8α-ergolinyl)-1,1-diethyl-harnstoff

Man löst 10 g 1,1-Diethyl-3-(6-methyl-8α-ergolinyl)-harnstoff (Tergurid) (30 mMol) in 550 ml Methylenchlorid und gibt unter Eiskühlung 8,8 ml 33 %ige Lösung von Bromwasserstoff in Eisessig zu. Unter weiterer Kühlung tropft man die Lösung von 3,1 ml Brom (60 mMol) in 400 ml Methylenchlorid innerhalb von 30 Minuten zu. Der Kristallbrei wird mit 200 ml Diisopropylether versetzt und die Kristalle nach 20 Minuten Rühren abfiltriert. Nach Umkristallisation aus Methylenchlorid, Methanol und Diisopropylether werden 10,5 g 3-(2,13-Dibrom-6-methyl-8α-ergolinyl)-1,1-diethyl-harnstoff erhalten. (71 % d.Th.).

$[\alpha]_D$ = + 1 ° (0,3 % in Methanol)

5.25 g dieser Verbindung (10,5 mMol) werden in 2,1 l Methanol gelöst, die Lösung auf - 20 °C abgekühlt und mit 15,7 g Cobaltchlorid (6 $H_2O$) versetzt. Dann gibt man unter weiterer Kühlung 3 g Natrimborhydrid in Tablettenform zu und rührt 1 Stunde lang. Die Reaktionslösung wird auf etwa 3 l Eis gegeben, mit 25 %iger Ammoniaklösung neutralisiert (0,5 Liter) und mit Methylenchlorid extrahiert. Die organische Phase wird mit Natriumsulfat getrocknet und eingedampft. Das Rohprodukt ergibt nach Kristallisation aus Essigester und Diisopropylether 3,5 g 3-(13-Brom-6-methyl-8α-ergolinyl)-1,1-diethyl-harnstoff (79 % d.Th.)

$[\alpha]_D$ = - 9° (0,5 % in Methanol)

Beispiel 6

3-(1-Acetyl-12-brom-2,3-dihydro-6-methyl-8α-ergolinyl)-1,1-diethyl-harnstoff

Eine Lösung von 5 g 3-(1-Acetyl-2,3-dihydro-6-methyl-8α-ergolinyl)-1,1-diethyl-harnstoff in 25 ml 5 %iger Essigsäure wird mit 1,0 ml Brom versetzt und die Mischung 35 Stunden bei Raumtemperatur gerührt. Die Reaktionsmischung wird auf 100 ml Eis gegossen, bis zur alkalischen Reaktion mit 25 %iger Ammoniaklösung versetzt und mit Dichlormethan extrahiert.

Die vereinigten organischen Phasen werden über Magnesiumsulfat getrocknet und eingeengt. Man erhält so 5,4 g 3-(1-Acetyl-12-brom-2,3-dihydro-6-methyl-8α-ergolinyl)-1,1-diethyl-harnstoff als Diastereomerengemisch, das chromatographisch an Kieselgel mit Dichlormethan/Ethanol im Verhältnis 95 : 5 aufgetrennt wird. Als Fraktion 1 werden 1,6 g und als Fraktion 2 1,5 g isoliert, die man zur vollständigen Reinigung aus Ethylacetat/Diisopropylether kristallisiert. Man erhält so 0,9 g 3-(1-Acetyl-12-brom-2,3α-dihydro-6-methyl-8α-ergolinyl)-1,1-diethyl-harnstoff (Fraktion 1)$[\alpha]_D$ = - 16 ° (0,5 % in Chloroform) und 0,8 g 3-(1-Acetyl-12-brom-2,3β-dihydro-6-methyl-8α-ergolinyl)-1,1-diethyl-harnstoff (Fraktion 2)$[\alpha]_D$ = + 28,4 ° (0,5 % in Chloroform).

Beispiel 7

3-(12-Brom-2,3α-dihydro-6-methyl-8α-ergolinyl)-1,1-diethyl-harnstoff

Eine Lösung von 1,8 g 3-(1-Acetyl-12-brom-2,3α-dihydro-6-methyl-8α-ergolinyl)-1,1-diethyl-harnstoff in 50 ml 1 n Salzsäure wird 4 Stunden bei 70 - 80 °C gerührt. Die Reaktionsmischung wird auf 100 ml Eis gegeben, bis zur alkalischen Reaktion mit 25 %iger Ammoniaklösung versetzt und mit Dichlormethan extrahiert. Die vereinigten organischen Phasen werden über Magnesiumsulfat getrocknet, eingeengt und der Rückstand an Kieselgel mit Dichlormethan/Methanol im Verhältnis 97 : 3 chromatographiert. Dabei werden 300 mg öliges Produkt isoliert, das man zur vollständigen Reinigung aus Ethylacetat/Diisopropylether kristallisiert. Man erhält so 200 mg 3-(12-Brom-2,3α-dihydro-6-methyl-8α-ergolinyl)-1,1-diethyl-harnstoff.

$[\alpha]_D$ = -6,5 ° (0,5 % in Chloroform)

Beispiel 8

3-(12-Brom-2,3β-dihydro-6-methyl-8α-ergolinyl)-1,1-diethyl-harnstoff

Analog Beispiel 7 werden aus 900 mg 3-(1-Acetyl-12-brom-2,3β-dihydro-6-methyl-8α-ergolinyl)-1,1-diethyl-harnstoff 500 mg öliges Produkt erhalten, das man zur vollständigen Reinigung aus Toluol/Pentan kristallisiert. Man erhält so 240 mg 3-(12-Brom-2,3β-dihydro-6-methyl-8α-ergolinyl)-1,1-diethyl-harnstoff.

$[\alpha]_D$ = + 50 ° (0,5 % in Chloroform).

Beispiel 9

3-(12-Brom-6-methyl-8α-ergolinyl)-1,1-diethyl-harnstoff

Eine Lösung von 4,2 g 3-(12-Brom-2,3-dihydro-6-methyl-8α-ergolinyl)-1,1-diethyl-harnstoff in einer Mischung aus 300 ml absolutem Tetrahydrofuran und 50 ml absolutem Triethylamin wird bei - 40 °C mit einer Lösung von 1,58 ml tert.-Butylhypochlorit in 250 ml absolutem Tetrahydrofuran versetzt und die Mischung 15 Minuten bei - 40 °C gerührt. Die Reaktionsmischung wird auf 1 Liter Eis gegossen, bis zur alkalischen Reaktion mit 25 %iger Ammoniaklösung versetzt und mit Dichlormethan extrahiert. Die vereinigten organischen Phasen werden über Magnesiumsulfat getrocknet und eingeengt. Das Rohprodukt wird aus Ethylacetat/Pentan kristallisiert und man erhält so 3 g 3-(12-Brom-6-methyl-8α-ergolinyl)-1,1-diethyl-harnstoff, $[\alpha]_D$ = - 0,3 ° (0,5 % in Chloroform).

Beispiel 10

3-(1-Acetyl-12-brom-9,10-didehydro-2,3-dihydro-6-methyl-8α-ergolinyl)-1,1-diethylharnstoff

Die Darstellung gelingt durch Umsetzung von 3-(1-Acetyl-9,10-didehydro-2,3-dihydro-6-methyl-8α-ergolinyl)-1,1-diethyl-harnstoff mit elementarem Brom in 5 %iger Essigsäure analog Beispiel 6.

Beispiel 11

3-(12-Brom-9,10-didehydro-2,3-dihydro-6-methyl-8α-ergolinyl)-1.1-diethyl-harnstoff

Diese Verbindung wird aus 3-(1-Acetyl-12-brom-9,10-didehydro-2,3-dihydro-6-methyl-8α-ergolinyl)-1,1-diethyl-harnstoff durch Erhitzen mit 1 n Salzsäure - analog Beispiel 7 - erhalten.

Beispiel 12

3-(12-Brom-9,10-didehydro-6-methyl-8α-ergolinyl)-1,1-diethyl-harnstoff

Die Darstellung erfolgt durch Umsetzung von 3-(12-Brom-9,10-didehydro-2,3-dihydro-6-methyl-8α-ergolinyl)-1,1-diethyl-harnstoff mit tert. Butylhypochlorit in Gegenwart von Triethylamin analog Beispiel 9.

Beispiel 13

3-(12-Brom-6-methyl-8α-ergolinyl)-1,1-diethylthioharnstoff

Man löst 6,29 g 3-(12-Brom-6-methyl-8α-ergolinyl)-1,1-diethyl-harnstoff (15 mMol) in einer Mischung aus 4,13 g frisch destilliertem Phosphoroxychlorid (45 mMol) und 50 ml wasserfreiem Methylenchlorid bei - 20 °C und läßt die Temperatur in 4 Stunden auf + 10 °C kommen. Über Nacht wird bei Raumtemperatur, dann noch 2 Stunden bei 40 °C gerührt und anschließend das Lösungsmittel in Vakuum abdestilliert. Der Rückstand wird in 50 ml wasserfreiem Acetonitril gelöst, auf - 10 °C abgekühlt, mit 7,2 g Kaliumethylxanthogenat (45 mMol) versetzt und 20 Stunden bei Raumtemperatur gerührt. Das Lösungsmittel wird weitgehend abdestilliert, dann verteilt man zwischen Ethylacetat und gesättigter Natriumcarbonatlösung, trocknet die organische Phase mit Natriumsulfat und dampft ein. Der Rückstand wird aus Ethylacetat kristallisiert und man erhält so 5,03 g (77 %) 3-(12-Brom-6-methyl-8α-ergolinyl)-1,1-diethylthioharnstoff.
$[\alpha]_D$ = + 55 ° (0,5 % in Chloroform)

Beispiel 14

3-(13-Brom-6-methyl-8α-ergolinyl)-1,1-diethylthioharnstoff

Analog Beispiel 13 werden 6,29 g 3-(13-Brom-6-methyl-8α-ergolinyl)-1,1-diethylharnstoff umgesetzt, aufbereitet und man erhält so 4,9 g (75 %) 3-(13-Brom-6-methyl-8α-ergolinyl)-1,1-diethylthioharnstoff.

**Patentansprüche**

1. 12- und 13-Brom-Ergolinderivate der allgemeinen Formel I

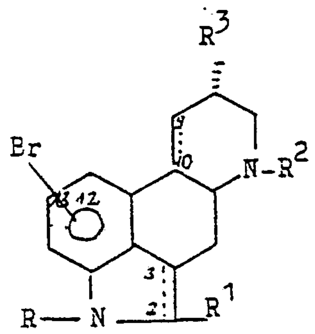

(I)

worin
R ein Wasserstoffatom oder eine Acetylgruppe,
$R^1$ ein Wasserstoffatom, Brom oder eine $C_{1-2}$-Akylthiogruppe, wobei R und $R^1$ nicht gleichzeitig ein Substituent sein kann,
$R^2$ eine $C_{1-4}$ - Alkylgruppe,
$R^3$ eine NH-CO-$NEt_2$-Gruppe oder eine NH-CS-$NEt_2$-Gruppe bedeutet und
$C_9$---$C_{10}$ und $C_2$---$C_3$ eine CC-Einfach- oder eine C=C-Doppelbindung bedeuten, wobei das Wasserstoffatom in 10-Stellung α-ständig ist, wenn $C_9$---$C_{10}$ eine CC-Einfacbindung ist und das Wasserstoffatom in 3-Stellung α-ständig oder β-ständig ist, wenn $C_2$---$C_3$ eine CC-Einfachbindung ist und falls Br in 12-Stellung steht, $R^1$ Wasserstoff bedeutet und falls Br in 2-Stellung steht $C_2$---$C_3$ eine C=C-Doppelbindung bedeutet und deren Säureadditionssalze.

**2.** 3-(13-Brom-6-methyl-2-methylthio-8α-ergolinyl)-1,1-diethylharnsoff

3-(13-Brom-6-methyl-8α-ergolinyl)-1,1-diethylharnstoff

3-(13-Brom-6-n-propyl-8α-ergolinyl)-1,1-diethylharnstoff

3-(13-Brom-2,3-dihydro-6-methyl-8α-ergolinyl)-1,1-diethylharnstoff

3-(2,13-Dibrom-6-methyl-8α-ergolinyl)-1,1-diethylharnstoff

3-(12-Brom-2,3α-dihydro-6-methyl-8α-ergolinyl-1,1-diethylharnstoff

3-(12-Brom-2,3ß -dihydro-6-methyl-8α-ergolinyl)-1,1-diethylharnstoff

3-(12-Brom-6-methyl-8α-ergolinyl)-1,1-diethylharnstoff

3-(12-Brom-9,10-didehydro-2,3 -dihydro-6-methyl-8α-ergolinyl)-1,1-diethylharnstoff

3-(12-Brom-9,10-didehydro-6-methyl-8α-ergolinyl)-1,1-diethylharnstoff

3-(12-Brom-6-methyl-8α-ergolinyl)-1,1-diethylthioharnstoff

3-(13-Brom-6-methyl-8α-ergolinyl)-1,1-diethylthioharnstoff

nach Anspruch 1.

**3.** Verfahren zur Herstellung der Verbindungen der allgemeinen Formel I, dadurch gekennzeichnet daß man eine Verbindung der allgemeinen Formel II

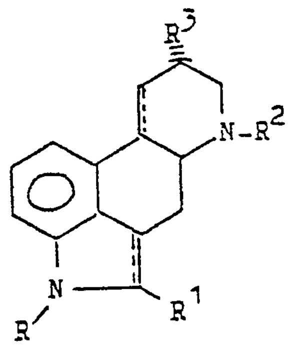

(II)

worin

R, $R^2$, $R^3$ und C---C die obige Bedeutung haben und $R^1$ Wasserstoff oder eine $C_{1-2}$-Alkylthiogruppe bedeutet, mit einem Bromierungsmittel umsetzt und anschließend gegebenenfalls in Verbindungen der allgemeinen Formel I mit einer $C_2=C_3$-Doppelbindung 2-Brom oder eine Alkylthiogruppe abspaltet und gewünscht enfalls die $C_2=C_3$ Doppelbindung hydriert oder in Verbindungen der allgemeinen Formel I mit einer $C_2$-$C_3$-Einfachbindung eine Acylgruppe abspaltet und gewünschtenfalls zur $C_2=C_3$-Doppelbindung dehydriert und anschließend gewünschtenfalls den Harnstoff in den Thioharnstoff überführt und gegebenenfalls das Säureadditionssalz bildet.

4. Verwendung der Verbindungen nach Anspruch 1 - 2 als Arzneimittel.

5. Arzneimittel auf Basis der Verbindungen nach den Ansprüchen 1 und 2.

6. Verwendung der Verbindungen nach Anspruch 1 - 2 zur Herstellung eines Arzneimittels zur Behandlung von psychischen Störungen.

## Claims

1. 12- and 13-bromo-ergoline derivatives of the general formula I

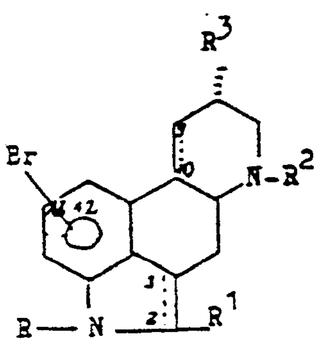

(I)

wherein

R represents a hydrogen atom or an acetyl group,

$R^1$ represents a hydrogen atom, bromine or a $C_{1-2}$alkylthio group in which R and $R^1$ cannot simultaneously be a substituent,

$R^2$ represents a $C_{1-4}$alkyl group,

$R^3$ represents a NH-CO-NEt$_2$ group or an NH-CS-NEt$_2$ group, and

$C_9$---$C_{10}$ and $C_2$---$c_3$ represent a CC single bond or a C=C double bond, wherein the hydrogen atom in the 10-position is in the $\alpha$-configuration when $C_9$---$C_{10}$ is a CC single bond and the hydrogen atom in the 3-position is in the $\alpha$-configuration or $\beta$-configuration when $C_2$---$C_3$ is a CC single bond and, if Br is in the 12-position, $R^1$ represents hydrogen and, if Br is in the 2-position, $C_2$---$C_3$ represents a C=C double bond, and the acid addition salts thereof.

**2.** 3-(13-Bromo-6-methyl-2-methylthio-8α-ergolinyl)-1,1-diethylurea

3-(13-bromo-6-methyl-8α-ergolinyl)-1,1-diethylurea

3-(13-bromo-6-n-propyl-8α-ergolinyl)-1,1-diethylurea

3-(13-bromo-2,3-dihydro-6-methyl-8α-ergolinyl)-1,1-diethylurea

3-(2,13-dibromo-6-methyl-8α-ergolinyl)-1,1-diethylurea

3-(12-bromo-2,3α-dihydro-6-methyl-8α-ergolinyl)-1,1-diethylurea

3-(12-bromo-2,3β-dihydro-6-methyl-8α-ergolinyl)-1,1-diethylurea

3-(12-bromo-6-methyl-8α-ergolinyl)-1,1-diethylurea

3-(12-bromo-9,10-didehydro-2,3-dihydro-6-methyl-8α-ergolinyl )-1,1-diethylurea

3-(12-bromo-9,10-didehydro-6-methyl-8α-ergolinyl)-1,1-diethylurea

3-(12-bromo-6-methyl-8α-ergolinyl)-1,1-diethylthiourea

3-(13-bromo-6-methyl-8α-ergolinyl)-1,1-diethylthiourea

according to claim 1.

**3.** Process for the preparation of compounds of the general formula I, characterised in that a compound of the general formula II

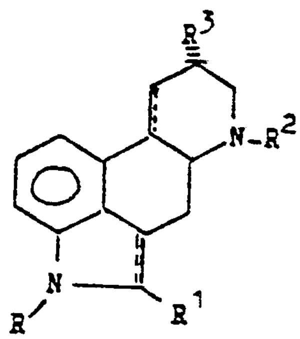

(II)

wherein
R, $R^2$, $R^3$ and C---C have the meaning given above and $R^1$ represents hydrogen or a $C_{1-2}$alkylthio group, is reacted with a brominating agent and then, optionally, in compounds of the general formula I having a $C_2=C_3$ double bond, 2-bromine or an alkylthio group is removed and, if desired, the $C_2=C_3$ double bond is hydrogenated or, in compounds of the general formula I having a $C_2$-$C_3$ single bond, an acyl group is removed and, if desired, dehydrogenated to the $C_2=C_3$ double bond and then, if desired, the urea is converted into the thiourea and optionally the acid addition salt is formed.

**4.** Use of the compounds according to claim 1-2 as medicaments.

**5.** Medicament based on compounds according to claims 1 and 2.

**6.** Use of compounds according to claims 1-2 for the manufacture of a medicament for the treatment of psychic disorders.

**Revendications**

**1.** Dérivés de la bromo-12 et de la bromo-13 ergoline qui répondent à la formule générale I :

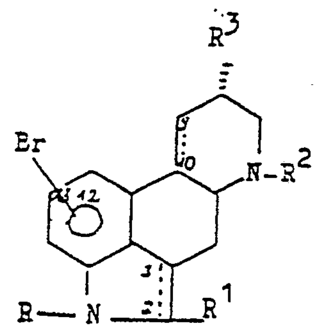

(I)

dans laquelle
R représente un atome d'hydrogène ou un radical acétyle,
$R^1$ représente un atome d'hydrogène, un atome de brome ou un radical alkylthio en $C_1$ ou $C_2$, avec la condition que R et $R^1$ ne peuvent pas représenter chacun en même temps un substituant,
$R^2$ représente un radical alkyle en $C_1$-$C_4$,
$R^3$ représente un radical -NH-CO-$NEt_2$ ou -NH-CS-$NEt_2$, et
$C_9$---$C_{10}$ et $C_2$---$C_3$ représentent chacun une liaison CC simple ou une double liaison C=C, l'atome d'hydrogène de la position 10 ayant la configuration α lorsque $C_9$---$C_{10}$ représente une liaison CC simple, et l'atome d'hydrogène de la position 3 ayant la configuration α ou β lorsque $C_2$---$C_3$ représente une liaison CC simple,
et dans laquelle, lorsque Br est en position 12, le symbole $R^1$ représente l'hydrogène et, lorsqu'il y a un Br à la position 2, l'ensemble $C_2$---$C_3$ représente une double liaison C=C,
ainsi que leurs sels d'addition d'acides.

**2.** Dérivés de l'ergoline selon la revendication 1, en l'espèce :
la (bromo-13 méthyl-6 méthylthio-2 ergolinyl-8α)-3 diéthyl-1,1 urée,
la (bromo-13 méthyl-6 ergolinyl-8α)-3 diéthyl-1,1 urée,
la (bromo-13 n-propyl-6 ergolinyl-8α)-3 diéthyl-1,1 urée,
la (bromo-13 dihydro-2,3 méthyl-6 ergolinyl-8α)-3 diéthyl-1,1 urée,
la (dibromo-2,13 méthyl-6 ergolinyl-8α)-3 diéthyl-1,1 urée,
la (bromo-12 dihydro-2,3α méthyl-6 ergolinyl-8α)-3 diéthyl-1,1 urée,
la (bromo-12 dihydro-2,3β méthyl-6 ergolinyl-8α)-3 diéthyl-1,1 urée
la (bromo-12 méthyl-6 ergolinyl-8α)-3 diéthyl-1,1 urée,
la (bromo-12 didéhydro-9,10 dihydro-2,3 méthyl-6 ergolinyl-8α)-3 diéthyl-1,1 urée,
la (bromo-12 didéhydro-9,10 méthyl-6 ergolinyl-8α)-3 diéthyl-1,1 urée,
la (bromo-12 méthyl-6 ergolinyl-8α)-3 diéthyl-1,1 thio-urée et
la (bromo-13 méthyl-6 ergolinyl-8α)-3 diéthyl-1,1 thio-urée.

**3.** Procédé pour préparer les composés de formule générale I, procédé caractérisé en ce qu'on fait réagir un composé répondant à la formule générale II :

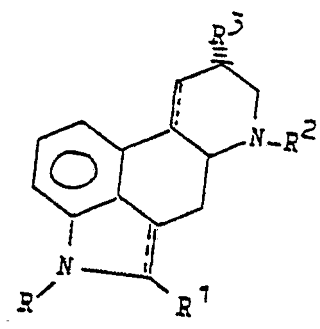

(II)

dans laquelle R, $R^2$, $R^3$ et C---C ont les significations qui leur ont été données ci-dessus, et $R^1$ représente l'hydrogène ou un radical alkylthio à 1 ou 2 atomes de carbone,
avec un agent de bromation, après quoi, le cas échéant, on élimine, dans des composés de formule générale I qui contiennent une double liaison $C_2=C_3$, l'atome de brome en 2 ou un radical alkylthio, et, si on le désire, on hydrogène la double liaison $C_2=C_3$, ou, dans des composés de formule générale I qui contiennent une liaison $C_2$-$C_3$ simple, on élimine un radical acyle et, si on le désire, on déshydrogène pour créer la double liaison $C_2=C_3$, puis, si on le désire, on transforme l'urée en thiourée et on forme éventuellement le sel d'addition avec un acide.

4. Application des composés selon l'une des revendications 1 et 2 comme médicaments.

5. Médicaments à base des composés selon l'une des revendications 1 et 2.

6. Application des composés selon l'une des revendications 1 et 2 à la fabrication d'un médicament destiné au traitement de troubles psychiques.